(19) Europäisches Patentamt / European Patent Office / Office européen des brevets

(11) **EP 2 906 683 B1**

(12) **EUROPEAN PATENT SPECIFICATION**

(45) Date of publication and mention
of the grant of the patent:
**31.05.2017 Bulletin 2017/22**

(21) Application number: **13824043.7**

(22) Date of filing: **14.10.2013**

(51) Int Cl.:
*C12N 5/00* (2006.01)    *C12N 15/62* (2006.01)
*C12N 15/63* (2006.01)    *C12P 21/02* (2006.01)
*C07K 14/705* (2006.01)    *C07K 16/28* (2006.01)

(86) International application number:
**PCT/US2013/064758**

(87) International publication number:
**WO 2014/062535 (24.04.2014 Gazette 2014/17)**

(54) **MAMMALIAN CELL CULTURE PROCESSES FOR PROTEIN PRODUCTION**

SÄUGERZELLKULTURVERFAHREN ZUR PROTEINPRODUKTION

PROCÉDÉS DE CULTURE DE CELLULES DE MAMMIFÈRES EN VUE DE LA PRODUCTION DE PROTÉINES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.10.2012 US 201261713812 P
09.10.2013 US 201361888647 P**

(43) Date of publication of application:
**19.08.2015 Bulletin 2015/34**

(73) Proprietor: **Bristol-Myers Squibb Company
Princeton, NJ 08543 (US)**

(72) Inventors:
• **TIAN, Jun**
  **East Syracuse, New York 13057 (US)**
• **BORYS, Michael**
  **Hopkinton, Massachusetts 01748 (US)**
• **LI, Zhengjian**
  **Hopkinton, Massachusetts 01748 (US)**
• **ABUABSI, Nicholas**
  **Hopkinton, Massachusetts 01748 (US)**
• **AU, Angela**
  **East Syracuse, New York 13057 (US)**
• **QIAN, Nan-xin**
  **East Syracuse, New York 13057 (US)**
• **DAI, Xiao-ping**
  **Flemington, NJ 08822 (US)**

(74) Representative: **Reitstötter Kinzebach
Patentanwälte
Sternwartstrasse 4
81679 München (DE)**

(56) References cited:
**WO-A1-2008/141207    WO-A1-2012/023085**

• **INN H. Y. YUK ET AL: "A GFP-based screen for growth-arrested, recombinant protein-producing cells", BIOTECHNOLOGY AND BIOENGINEERING, vol. 79, no. 1, 5 July 2002 (2002-07-05), pages 74-82, XP055105502, ISSN: 0006-3592, DOI: 10.1002/bit.10293**
• **JONG KWANG HONG ET AL: "Growth factor withdrawal in combination with sodium butyrate addition extends culture longevity and enhances antibody production in CHO cells", JOURNAL OF BIOTECHNOLOGY, ELSEVIER SCIENCE PUBLISHERS, AMSTERDAM, NL, vol. 155, no. 2, 17 June 2011 (2011-06-17) , pages 225-231, XP028260117, ISSN: 0168-1656, DOI: 10.1016/J.JBIOTEC.2011.06.020 [retrieved on 2011-06-23]**
• **BRIAN RASMUSSEN ET AL: "Isolation, characterization and recombinant protein expression in Veggie-CHO: A serum-free CHO host cell line", CYTOTECHNOLOGY, KLUWER ACADEMIC PUBLISHERS, DO, vol. 28, no. 1-3, 1 September 1998 (1998-09-01), pages 31-42, XP019236568, ISSN: 1573-0778, DOI: 10.1023/A:1008052908496**

**(Cont. next page)**

- **MORRIS A E ET AL: "EFFECTS OF INSULIN AND LONGR3 ON SERUM-FREE CHINESE HAMSER OVARY CELL CULTURES EXPRESSING TWO RECOMBINANT PROTEINS", BIOTECHNOLOGY PROGRESS, AMERICAN INSTITUTE OF CHEMICAL ENGINEERS, US, vol. 16, no. 5, 1 January 2000 (2000-01-01), pages 693-697, XP000979091, ISSN: 8756-7938, DOI: 10.1021/BP0000914**
- **SUNLEY K ET AL: "Strategies for the enhancement of recombinant protein production from mammalian cells by growth arrest", BIOTECHNOLOGY ADVANCES, ELSEVIER PUBLISHING, BARKING, GB, vol. 28, no. 3, 1 May 2010 (2010-05-01), pages 385-394, XP026983670, ISSN: 0734-9750 [retrieved on 2010-02-13]**
- **NATALIA GOMEZ ET AL: "Culture temperature modulates aggregation of recombinant antibody in cho cells", BIOTECHNOLOGY AND BIOENGINEERING., vol. 109, no. 1, 1 January 2012 (2012-01-01), pages 125-136, XP055271209, US ISSN: 0006-3592, DOI: 10.1002/bit.23288**

**Description**

FIELD OF THE INVENTION

The present invention relates to new processes for culturing CHO cells which produce a protein product. Performance of the cell culturing processes result in high cell viability and/or cell density and can also result in high product quality and productivity.

BACKGROUND OF THE INVENTION

Animal cell culture, notably mammalian cell culture, is preferably used for the expression of recombinantly produced proteins for therapeutic and/or prophylactic applications.

In general, protein expression levels in mammalian cell culture-based systems are considerably lower than in microbial expression systems, for example, bacterial or yeast expression systems. However, bacterial and yeast cells are limited in their ability to optimally express high molecular weight protein products, to properly fold a protein having a complex steric structure, and/or to provide the necessary post-translational modifications to mature an expressed glycoprotein, thereby affecting the immunogenicity and clearance rate of the product.

As a consequence of the limitations of the culturing of animal or mammalian cells, particularly animal or mammalian cells which produce recombinant products, the manipulation of a variety of parameters has been investigated, including the employment of large-scale culture vessels; altering basic culture conditions, such as incubation temperature, dissolved oxygen concentration, pH, and the like; the use of different types of media and additives to the media; and increasing the density of the cultured cells. In addition, process development for mammalian cell culture would benefit from advances in the ability to extend run times to increase final product concentration while maintaining high product quality. Run times of cell culture processes, particularly non-continuous processes, are usually limited by the remaining viability of the cells, which typically declines over the course of the run. The maximum possible extension of high cell viabilities is therefore desired while maintaining product quality. Protein purification concerns offer yet another motivation for minimizing decreases in viable cell density and maintaining high cell viability. The presence of cell debris and the contents of dead cells in the culture can negatively impact on the ability to isolate and/or purify the protein product at the end of the culturing run. By keeping cells viable for a longer period of time in culture, there is thus a concomitant reduction in the contamination of the culture medium by cellular proteins and enzymes that can cause degradation and ultimate reduction in quality of the desired protein produced by the cells.

Various parameters have been investigated to achieve high cell viability in cell cultures. One parameter involved a single lowering of the culture temperature following initial culturing at 37 °C (for example, Roessler et al., Enzyme and Microbial Technology, 18:423-427 (1996); U.S. Patent Nos. 5,705,364 and 5,721,121 to Etcheverry, T. et al. (1998); U.S. Patent No. 5,976,833 to Furukawa, K. et al. (1999); U.S. Patent No. 5,851,800 to Adamson, L. et al.; WO 99/61650 and WO 00/65070 to Genentech, Inc.; WO 00/36092 to Biogen, Inc.; and U.S. Patent No. 4,357,422 to Girard et al.).

Other parameters investigated involved the addition of components to the culture. The addition of dextran sulfate and polyvinyl sulfate to CHO 111-10PF cell line was found to increase day 3 viable cell density and viability relative to the control culture (Zhangi et al., Biotechnol. Prog., 16:319-325 (2000)). The effect of dextran sulfate or polyvinyl sulfate during the death phase was however not reported. Dextran sulfate and polyvinyl sulfate were also reported to be effective at preventing cell aggregation.

Protein therapeutics are inherently heterogeneous owing to their size, complexity of structure, and the nature of biological production (Chirino et al., Nat. Biotechnol., 22:1383-1391 (2004)). Even in the "pure" protein solution, there will be some percentage of low molecular weight fragments, high molecular weight species, and various degrees of chemical modifications. The formation of high molecular weight species is usually due to protein aggregation, which is a common issue encountered during manufacture of biologics. Typically, the presence of aggregates is considered to be undesirable because of the concern that the aggregates may lead to an immunogenic reaction or may cause adverse events on administration (Cromwell et al., AAPSJ., 8:E572-E579 (2006)). Although some types of aggregates of biologics may function normally, it is still important to maintain consistency in product quality since product consistency is a prerequisite for regulatory approval.

Aggregates of proteins may arise from several mechanisms and occur at each stage during the manufacturing process. In cell culture, secreted proteins may be exposed to the conditions that are unfavorable for protein stability; but more often, accumulation of high amounts of protein may lead to intracellular aggregation owing to either the interactions of unfolded protein molecules or to inefficient recognition of the nascent peptide chain by molecular chaperones responsible for proper folding (Cromwell et al., AAPS J., 8:E572-E579 (2006)). In the endoplasmic reticulum (ER) of cells, disulfide bond of newly synthesized protein is formed in an oxidative environment. Under normal condition, protein sulfhydryls are reversibly oxidized to protein disulfides and sulfenic acids, but the more highly oxidized states such as the sulfinic and sulfonic acid forms of protein cysteines are irreversible (Thomas et al., Exp. Gerontol., 36:1519-1526 (2001)). Hyper-oxidized proteins may contain incorrect disulfide bonds or have mixed disulfide bonds with other luminal ER proteins; in either case it leads to protein improper folding and aggregation. It is therefore crucial to maintain a properly controlled oxidative environment in the ER. In this regard, Cuozzo et al. (Nat. Cell Biol., 1:130-135 (1999)) initially demonstrated that in yeasts glutathione buffered against ER hyperoxidation and later on Chakravarthi et al. (J. Biol. Chem., 279:39872-39879 (2004)) confirmed that in mammalian cells glutathione was also required to regulate the formation of native disulfide bonds within proteins entering the secretory pathway. It has been described that culture temperature modulates aggregation of a recombinant antibody in CHO cells (Gomez et al. (Biotechnol. Bioeng. 109:125-136 (2012))

With increasing product concentration in the culture, it can be observed in cell culture processes that the product quality decreases. High abundance of a protein produced by cells in culture is optimally accompanied by high quality of the protein that is ultimately recovered for an intended use.

Recombinantly produced protein products are increasingly becoming medically and clinically important for use as therapeutics, treatments and prophylactics. Therefore, the development of reliable cell culture processes that economically and efficiently achieve an increased final protein product concentration, in conjunction with a high level of product quality, fulfills both a desired and needed goal in the art.

SUMMARY OF THE INVENTION

The present invention relates to a method of decreasing aggregation of a protein of interest during cell culture production phase in CHO cells, comprising adapting CHO cells which express the protein of interest to growth factor, protein and peptide free media; and culturing the adapted CHO cells in growth factor, protein and peptide free media. Accordingly, new processes for the production of proteins by CHO cell cultures are disclosed herein. These new processes achieve increased viable cell density, cell viability, productivity and decreased protein aggregation.

One aspect of the disclosure concerns the growth of cells in a growth factor/protein/peptide free media throughout the culture process. In this aspect, cell culture processes of this disclosure can advantageously achieve an enhanced specific productivity of the protein produced by the cultured cells. More specifically, in accordance with this disclosure, growth factor/protein/peptide free media utilized during the cell culturing period sustains a high cell viability of the cells in the culture and can provide a high quantity and quality of produced product throughout an entire culture run. Also, according to one aspect of this disclosure, growth factor/protein/peptide free media utilized during the culturing processes can advantageously allow for an extension of the production phase of the culture. During the extended production phase, the titer of the desired product is increased; the product quality is maintained at a high level; protein aggregation level is maintained at lower level and cell viability is also maintained at a high level. In addition, the extended production phase associated with the culturing processes allows for the production of product beyond that which is produced during a standard production phase.

Another aspect of this disclosure concerns the addition of one or more growth factors to the growth factor/protein/peptide free cell culture after inoculation. In accordance with this disclosure, addition of one or more growth factors to the growth factor/protein/peptide free cell culture after inoculation sustains a high cell viability of the cells in the culture and can provide a high quantity and quality of produced product throughout an entire culture run. During the production phase of the growth factor feed cell culture, the titer of the desired product is increased; the product quality is maintained at a high level; protein aggregation level is maintained at lower level and cell viability is also maintained at a high level.

In one particular aspect, the present disclosure provides a process (or method) in which the protein aggregation level was reduced by the addition of insulin and/or IGF in the fed medium. In accordance with this particular aspect, the addition of insulin and or IGF sustains a high cell viability of the culture, thereby, the titer of product, preferably recombinant product, is increased and the product quality is maintained at high level.

In one aspect of this disclosure, one or more growth factors are added to a culture at the time of inoculation or at a time after inoculation that is before the beginning of the initial death phase, or is during the initial growth phase, or is during the second half of the initial growth phase, or is on or about the end of the initial growth phase. In accordance with this aspect of the disclosure, the growth phase is extended and/or the onset of the death phase is delayed for a period of time, such as several days.

Further aspects, features and advantages of the present invention will be appreciated upon a reading of the detailed description of the invention and a consideration of the drawings/figures.

DESCRIPTION OF THE DRAWINGS/FIGURES

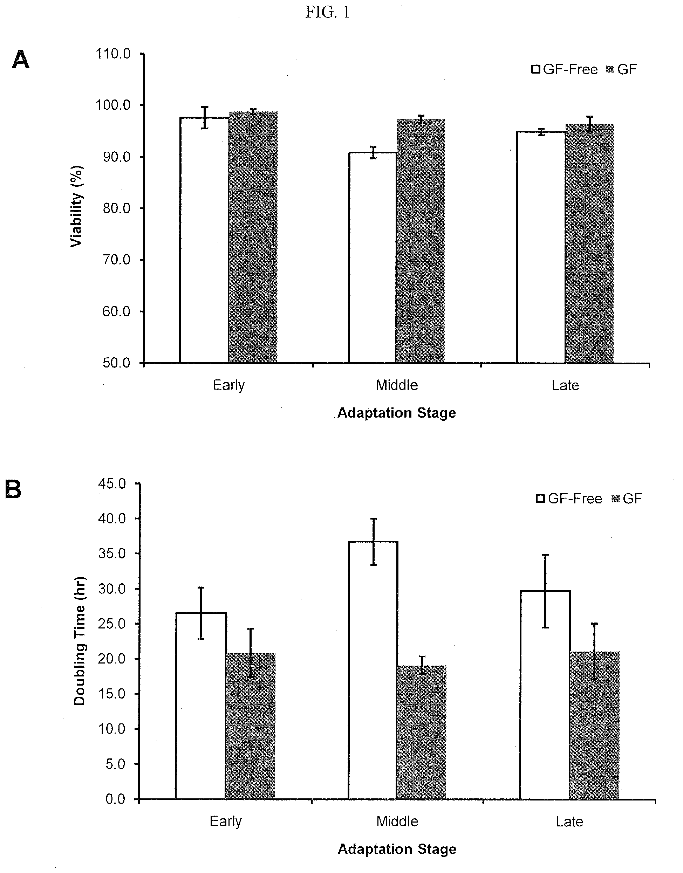

Figure 1A and 1B shows the adaptation of CHO cells (Clone A) to growth in medium without growth factors (GF). As described in Example 1, cells were passaged every 3 or 4 days in medium with (filled bar; GF) or without (empty bar; GF-free) growth factors (GF). (A) Cell viability and (B) doubling time at different stages of adaptation. Early: passage 1 to 4; middle: passage 5 to 8; late: passage 9 to 12.

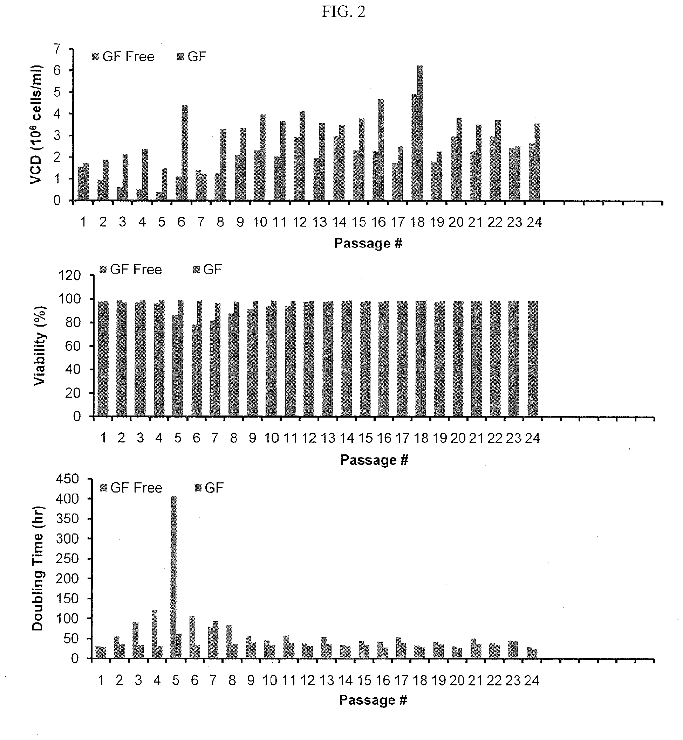

**Figure** 2 shows viable cell density (VCD), percent viability and doubling time for **Clone B.** The CHO cell line used in this study was originally subcloned from DG44 parental cells and cultured in a growth factor/protein/peptide free basal medium (GF-free) or basal medium with the growth factor (GF) insulin, as described in Example 1.

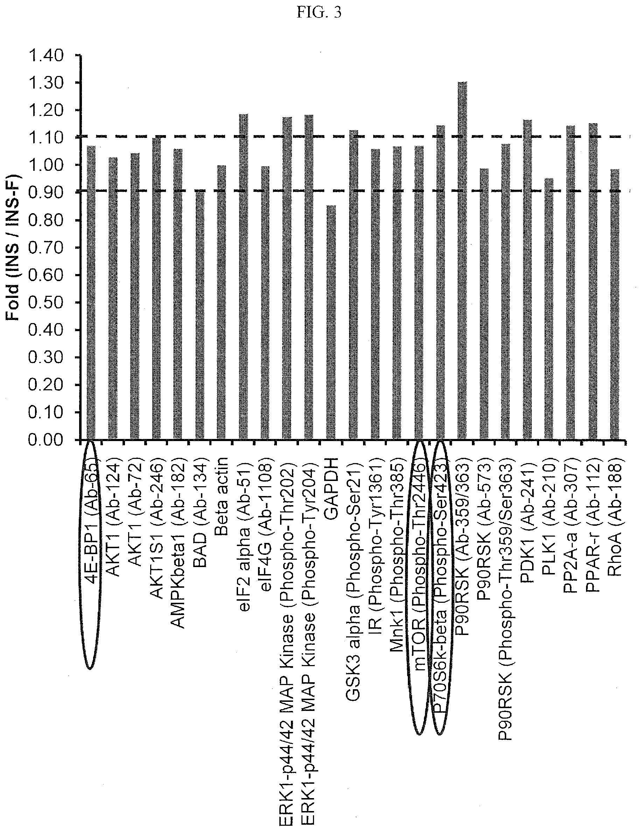

**Figure 3** shows the change in expression/phosphorylation of proteins from the mTOR pathway when **Clone A** cells are grown with (INS) and without insulin (INS-F), as described in Example 2.

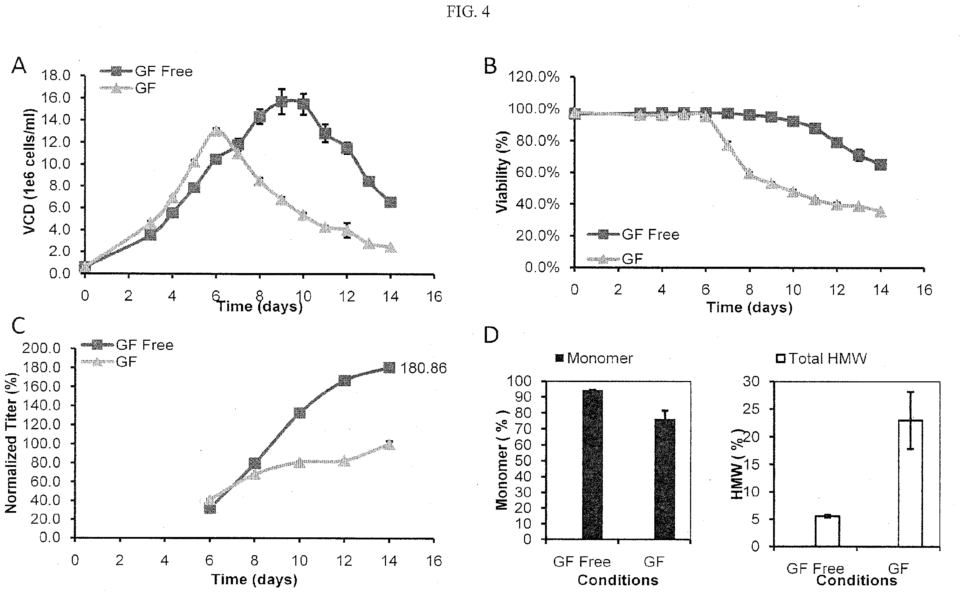

**Figure 4A-D** shows viable cell density (VCD), percent viability, aCD40L protein titer, percent aCD40L monomer for **Clone A** grown in basal medium with 1mg/L insulin and 10mg/L in the feed medium (INS) or without insulin in the basal or feed medium(INS-Free) as described in Example 3.

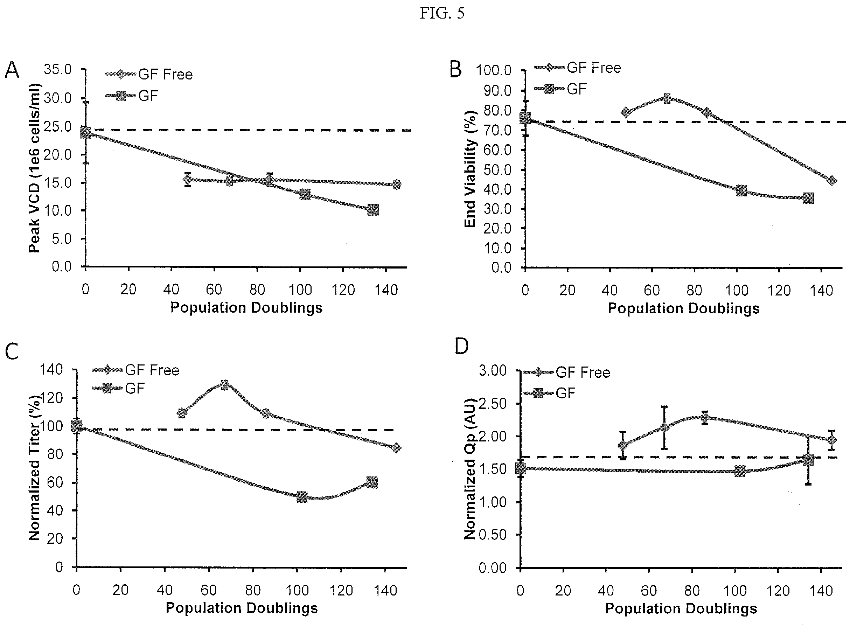

**Figure 5A-D** shows GF-free and GF cells at different ages or population doublings evaluated using a fed-batch culture approach of **Clone A.** Compared were factors including the peak viable cell density (A), the end viability (B), the normalized protein titer (C), and the normalized specific productivity (D) for the fed-batch cultures as described in Example 3. Note that GF-free cells maintained the factors relatively stable comparing to those for the GF cells.

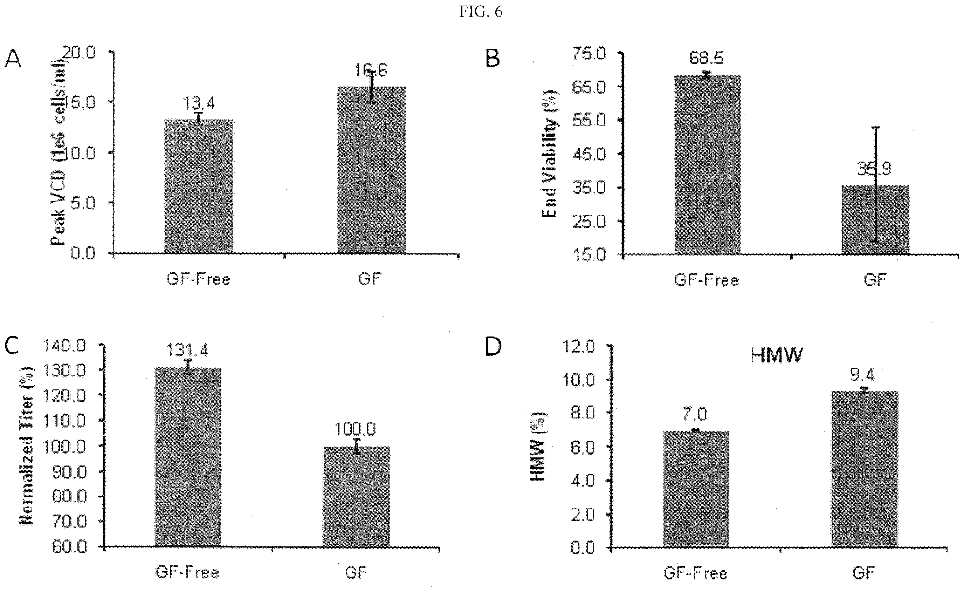

**Figure 6A-D** shows GF-free culture improved the end viability and protein titer, and reduced the high molecular species for **Clone B.** Cells adapted to grow in medium without growth factors (GF-Free) were at similar passage number as the cells always exposed to insulin (GF). The GF-Free and GF cells were cultured in fed-batch mode following the same process as described in Example 3. Both the basal medium and feed medium used were chemically defined. (A) peak viable cell density (Peak VCD), (B) end cell viability, and (C) normalized protein titer on day 14 for the fed-batch culture process. (D) total high molecular species (HMW) for the protein produced from GF-Free and GF cells at the end of the culture (day 14).

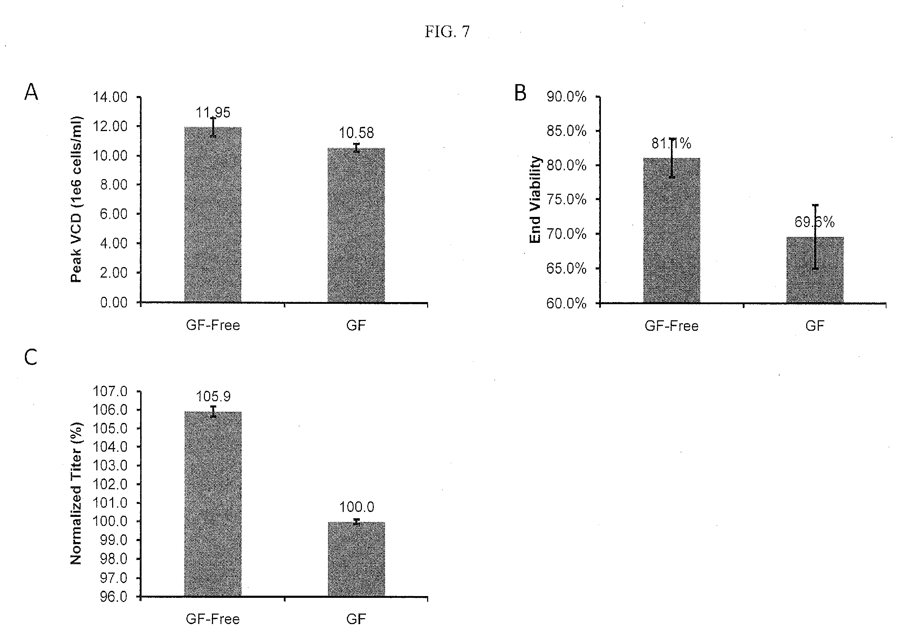

**Figure 7A-C** shows GF-free culture improved the cell growth, end viability, and protein titer for **Clone C.** Cells adapted to grow in medium without growth factors (GF-free) were at similar passage number as the cells always exposed to insulin (GF). The GF-free and GF cells were cultured in batch mode following the same process as described in Example 3. The medium used for the culture was chemically defined. (A) peak viable cell density (Peak VCD), (B) end cell viability, and (C) normalized protein titer for the batch culture process.

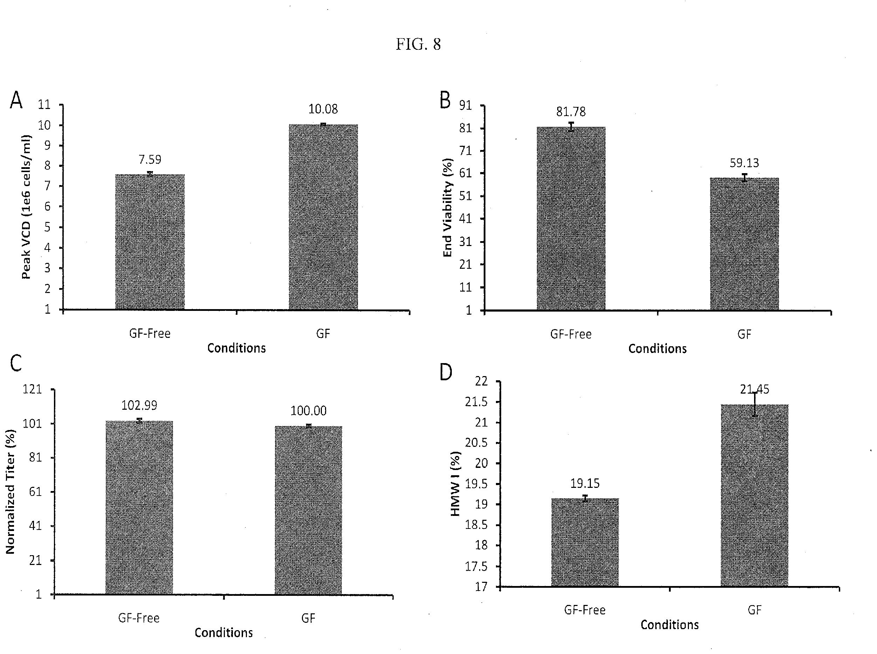

**Figure 8A-D** shows GF-free culture improved end viability and reduced high molecular species for **Clone D.** Cells adapted to grow in medium without growth factors (GF-free) were at similar passage number as the cells always exposed to insulin (GF). The GF-free and GF cells were cultured in a fed-batch mode following the same process as described in Example 3. The basal medium and feed medium used for the culture were chemically defined. (A) peak viable cell density (Peak VCD), (B) end cell viability, and (C) normalized protein titer on day 14 for the fed-batch culture process. (D) total high molecular species (HMW) for the protein produced from GF-free and GF cells at the end of the culture (day 14).

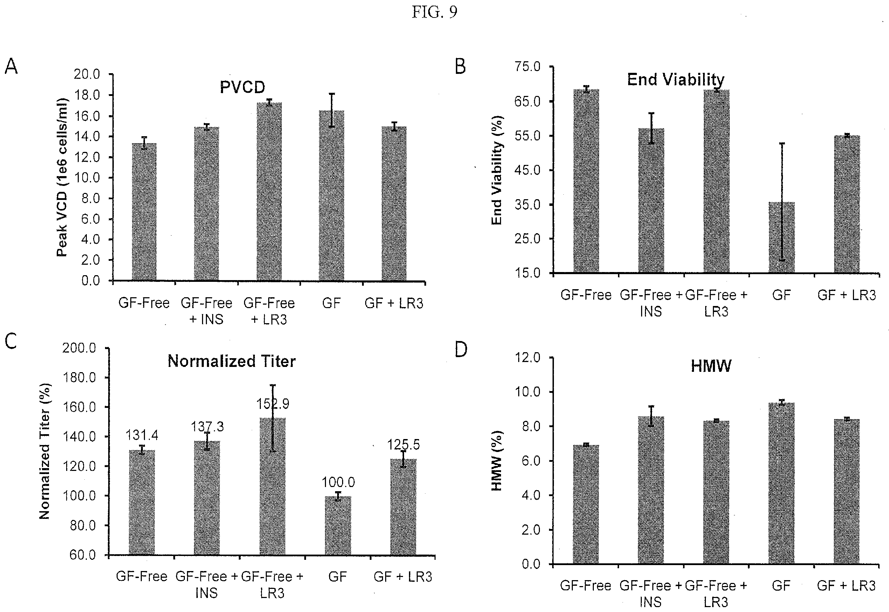

**Figure 9A-D** shows GFs added back to GF-free culture improved cell growth, end viability, and protein titer for **Clone B.** Cells adapted to grow in medium without growth factors (GF-Free) were at similar passage number as the cells always exposed to insulin (GF). The GF-Free and GF cells were cultured in a fed-batch mode in chemically defined basal and feed media with or without GFs as described in Example 4. GF-Free: no GFs were present in cell culture media; GF-Free + INS: GF-Free cells cultured in basal medium with 1 mg/L insulin and in feed medium with 10 mg/L insulin; similarly, GF-Free + LR3: 4 $\mu$g/L LONG®R3 (LR3) in basal medium and 40 $\mu$g/L LR3 in feed medium; GF: 1 mg/L insulin in basal medium and 10 mg/L insulin in feed medium; GF + LR3: 1 mg/L insulin and 4 $\mu$g/L LR3 in basal medium and 10 mg/L insulin and 40 $\mu$g/L LR3 in feed medium. (A) peak viable cell density (Peak VCD), (B) end cell viability, and (C) normalized protein titer for the cells cultured in a fed-batch mode in the aforementioned conditions. (D) total high molecular species (HMW) for the protein produced under the designated conditions at the end of the culture (day 14).

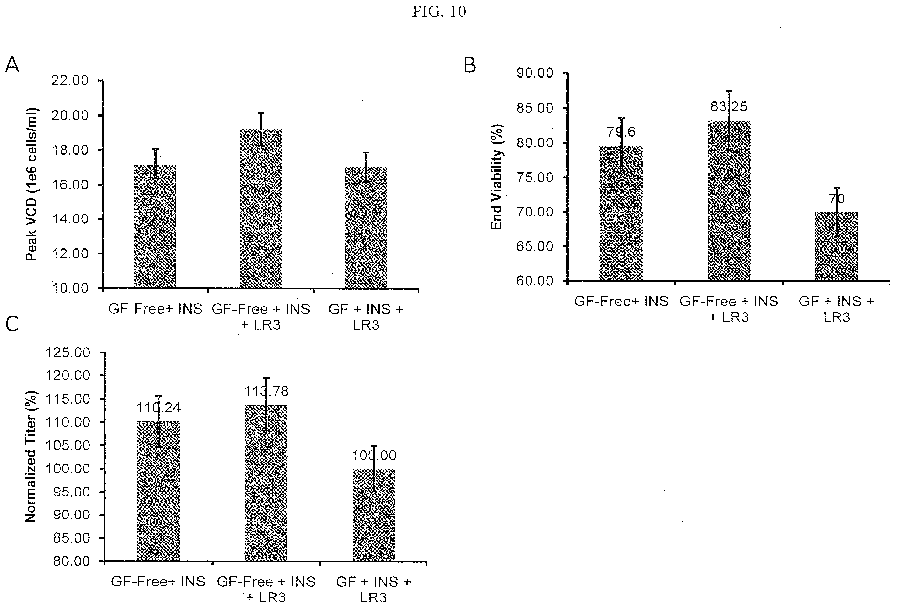

**Figure 10A-C** shows GFs added back to GF-free culture improved cell growth, end viability, and protein titer for **Clone C.** Cells adapted to grow in medium without growth factors (GF-Free) were at similar passage number as the cells always exposed to insulin (GF). The GF-Free and INS cells were cultured in a fed-batch mode in chemically defined basal and feed media with or without GFs as described in Example 4. GF-Free + INS: GF-Free cells cultured in basal medium with 1 mg/L insulin and in feed medium with 10 mg/L insulin; similarly, GF-Free + INS + LR3: 1 mg/L insulin and 4 $\mu$g/L LR3 in basal medium and 10 mg/L insulin and 40 $\mu$g/L LR3 in feed medium; GF + INS + LR3: GF cells cultured in the presence of 1 mg/L insulin and 4 $\mu$lg/L LR3 in basal medium and 10 mg/L insulin and 40 $\mu$g/L LR3 in feed medium. (A) peak viable cell density (Peak VCD), (B) end cell viability, and (C) normalized protein titer for the cells cultured in a fed-batch mode in the aforementioned conditions.

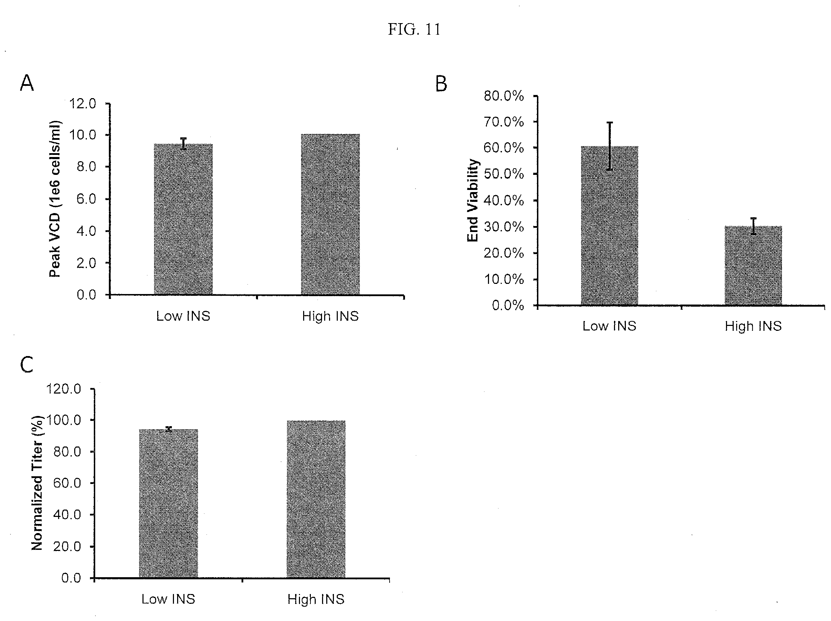

**Figure 11A-C** shows GFs added back to cultures with lower GF concentration improved end viability, and maintained cell growth and protein titer for **Clone C.** Cells adapted to grow in medium with lower insulin concentration (0.01 mg/L; Low INS) were at similar passage number as the cells always cultured in the same medium with higher insulin concentration (1 mg/L; High INS). Using the same fed-batch process as described in Example 4, the Low INS cells were cultured in basal medium with 0.01 mg/L insulin, and in feed medium with10 mg/L insulin; the High INS cells were cultured in basal medium with 1 mg/L insulin, and in feed medium with 10 mg/L insulin. (A) peak viable cell density (Peak VCD), (B) end cell viability, and (C) normalized protein titer on day 14 for the cells cultured in the aforementioned conditions.

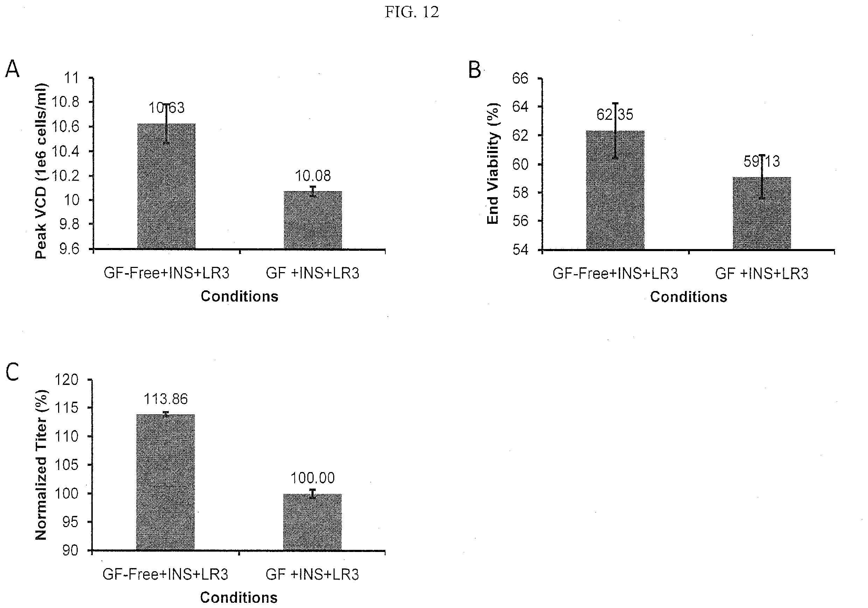

**Figure 12A-C** shows GFs added back to GF-free culture improved end viability and protein titer for **Clone D.** Cells adapted to grow in medium without growth factors (GF-Free) were at similar passage number as the cells always exposed to insulin (1 mg/L; GF). The GF-Free and GF cells were cultured in a fed-batch mode in chemically defined basal and feed media with or without GFs as described in Example 4. GF-Free + INS + LR3: GF-Free cells cultured in basal medium without GFs and in feed medium with 10 mg/L insulin and 40 μg/L LONG®R3 (LR3); similarly, GF + INS + LR3: GF cells cultured in the presence of 1 mg/L insulin in basal medium and 10 mg/L insulin and 40 μg/L LR3 in feed medium. (A) peak viable cell density (Peak VCD), (B) end cell viability, and (C) normalized protein titer on day 14 for the cells cultured in the aforementioned conditions.

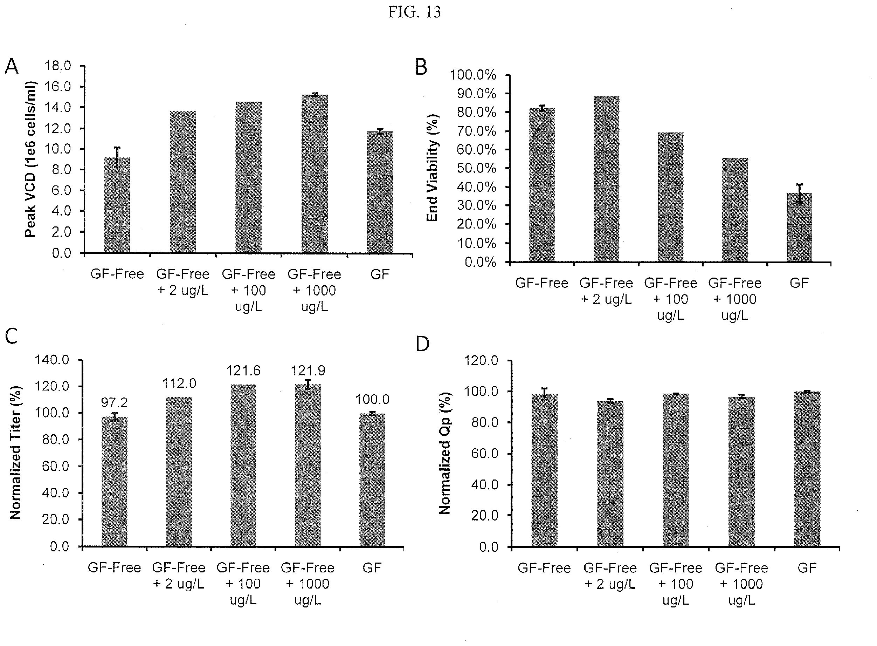

**Figure 13A-D** shows GF add-back strategy minimizes the GF concentration required for similar process performance. **Clone B** cells adapted to grow in medium without growth factors (GF-Free) were at similar passage number as the cells always exposed to insulin (1 mg/L; GF) as described in Example 4. With the same process the GF-Free cells were cultured in a fed-batch mode in a chemically defined basal medium with different concentrations of insulin: 0 mg/L (GF-Free), 0.002 mg/L (GF-Free + 2 μg/L), 0.1 mg/L (GF-Free +100 μg/L), or 1 mg/L (GF-Free + 1000 μg/L). GF cells were cultured in basal medium with 1 mg/L insulin. The insulin concentration in the feed medium is 10 fold of that in the basal medium for the corresponding conditions. (A) peak viable cell density (Peak VCD), (B) end cell viability, (C) normalized protein titer on day 14, and (D) normalized specific productivity (Qp) for the cells cultured in the aforementioned conditions.

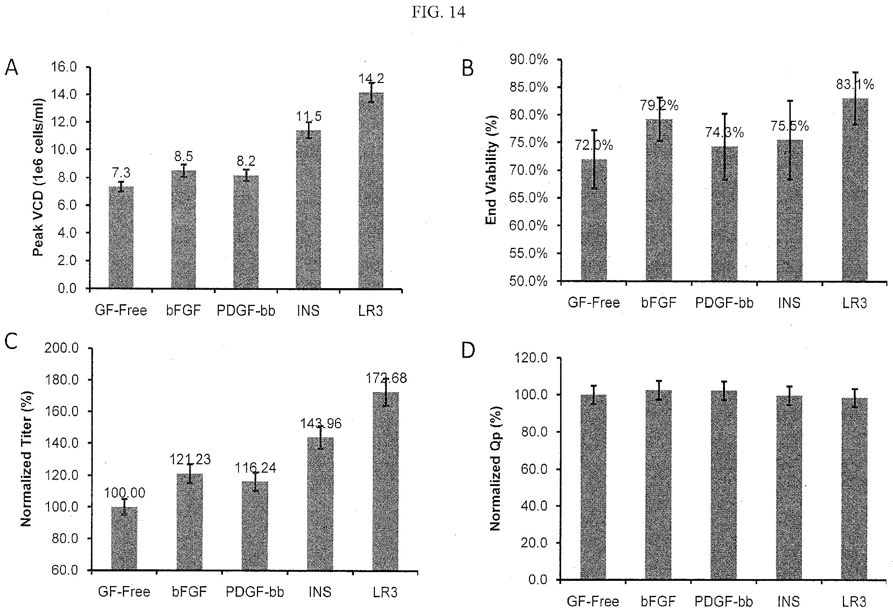

**Figure 14A-D** shows different GFs can be added back to GF-free cultures to boost cell growth and protein titer. **Clone B** cells were adapted to grow in medium without growth factors (GF-Free) as described in Example 4. The adapted GF-Free cells were cultured in a fed-batch mode in chemically defined basal and feed media without (GF-Free) or with different GFs at 4 μg/L. bFGF: basic fibroblast growth factor; PDGF-bb: Platelet-derived growth factor, two B chains; INS: insulin; LR3: LONG®R3, a recombinant IGF1 analog. The concentration for GFs in the feed medium is 5 fold of that in the basal medium for the corresponding conditions. (A) peak viable cell density (Peak VCD), (B) end cell viability, (C) normalized protein titer on day 14, and (D) normalized specific productivity (Qp) for the cells cultured in the aforementioned conditions.

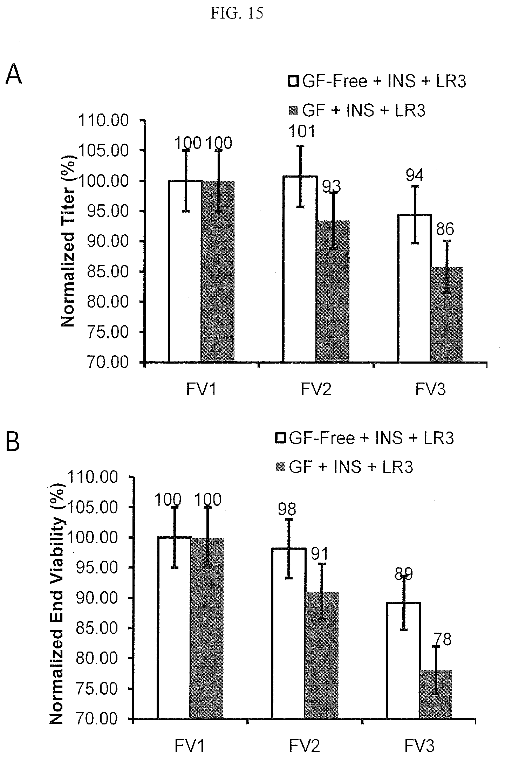

**Figure 15A-B** shows GF add-back strategy improved process robustness for Clone C. Cells adapted to grow in medium without growth factors (GF-Free) were at similar passage number as the cells always exposed to insulin (1 mg/L; GF). The GF-Free and INS cells were cultured in a fed-batch mode in chemically defined basal and feed media as described in Example 4. The basal medium for GF-Free cells was absent of GFs, and for INS cells contained 1 mg/L insulin. Different feeding volume were applied for each condition on a daily basis beginning on day 3: FV1: feeding volume (FV) at 3.6%; FV2: 4.3%; and FV3: 5% of initial volume. The feed medium contained both insulin and LR3 for each condition. For FV1: insulin was at 10 mg/L, and LR3 at 42 μg/L. The concentration for insulin and LR3 in FV2 and FV3 was adjusted so that same amount (grams) of insulin and LR3 were added to the culture vessel, although the feeding volume was different. (A) normalized protein titer on day 14, and (B) normalized end viability for the cells cultured in the aforementioned conditions. Note that the GF-Free cells maintained the end viability and protein titer relatively stable against the process perturbation (increase in feeding volume).

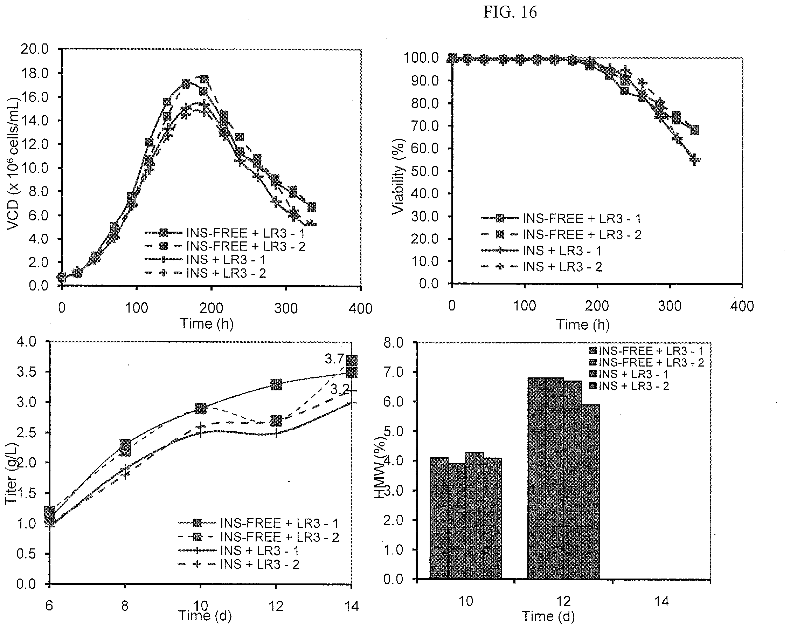

**Figure 16** shows viable cell density (VCD), percent viability, aCD40L protein titer, percent aCD40L high molecular weight (HMW) for **Clone B** grown in a 5L reactor where the growth factors insulin (INS) and/or LONG®R3 (LR3) are added back to the basal and feed media after inoculation as described in Example 5.

DETAILED DESCRIPTION OF THE INVENTION

The present invention describes new processes for the production of proteins, preferably recombinant protein products, in CHO cell culture. These processes achieve increased viable cell density, cell viability, productivity and decreased protein aggregation.

Chinese hamster ovary (CHO) cells have been one of the major cell types used for the production of recombinant therapeutics. For the large-scale manufacturing of such therapeutics in CHO cells with chemically defined cell culture media, growth factors (GF) are widely used to promote cell growth and productivity. However, the use of growth factors not only significantly increases the cost of manufacturing, but potentially also impacts the process performance and robustness due to complicated cell signaling from the growth factors.

The table below describes the clones utilized to exemplify the invention. Each clone is a CHO cell line with a DHFR selection system expressing a fusion protein.

|  | A | B | C | D |
|---|---|---|---|---|
| Clone | 40A6 | 63C2 | D7 | Aba |
| Molecule | fusion protein-1 | fusion protein-1 | fusion protein-2 | fusion protein-3 |
| Cell Line | CHO | CHO | CHO | CHO |
| Selection System | DHFR | DHFR | DHFR | DHFR |

Cells Grown Under Growth Factor-Free Cell Culture Conditions

One embodiment of the invention shows that signaling from growth factors could be removed for CHO cell culture. Example 1 shows that two different dihydrofolate reductase (DHFR) clones expressing a domain fusion antibody, can be adapted to growth factor free (GF-free) media by continuously culturing them in the media for up to nine passages(see Figures 1 and 2). Interestingly, as shown in Example 2, the mTOR pathway, which regulates cell growth and protein production, was not significantly impacted by growth factor removal as shown by antibody arrays that target 138 proteins involved in the mTOR pathway(see Figure 3).

Production assays of fed-batch cultures described in Example 3 found that the GF-free condition increased peak viable cell density to $15.7 \times 10^6$ cells/ml from $13.0 \times 10^6$ cells/ml for the GF condition, and improved the cell viability at the later stages of the culture, from 59.4% for the GF condition to 96.2% on day 8, and from 35.6% to 65.1% on day 14. As a result, the protein titer was increased by 80.9%. In addition, the protein quality was enhanced under the GF-free condition as the high molecular species decreased to 4.8% from 14.3% for the GF condition(see Figure 4).

Additionally, Figures 5 - 8 show that the production stability of the cells was improved under the GF-free condition in three different clones. The productivity decreased after cells were cultured under the GF condition, while cells cultured under GF-free condition maintained their productivity.

Overall, our results demonstrate that chemically defined medium free of proteins and peptides is capable of propagating CHO cells, delivering productivities comparable to the GF condition, and better maintaining the production stability of clones.

It has been found (see Figures 4, 6, 7 and 8), that growing cells in growth factor-free conditions increases cell viability at later stages of the culture and increases protein titer, while reducing aggregation of the proteins of interest.

Disclosed herein is thus a cell culturing process for increasing cell viability at later stages of culture comprising culturing host cells, which express a protein of interest; in growth factor/protein/peptide free media, wherein the end stage cell viability is increased compared to end stage cell viability in cells grown in media comprising growth factor/protein/peptide.

Further disclosed herein is a cell culturing process for increasing production of a protein of interest comprising culturing host cells, which express a protein of interest; in growth factor/protein/peptide free media, wherein the protein titer is increased compared to protein titer in cells grown in media comprising growth factor/protein/peptide.

Further disclosed herein is a cell culturing process for reducing the percentage of protein aggregation comprising: culturing host cells, which express a protein of interest; in growth factor/protein/peptide free media, wherein the percentage of high molecular weight species is decreased compared to the percentage of high molecular weight species in cells grown in media comprising growth factor/protein/peptide.

Growth Factor Add-Back to Growth Factor-Free Cell Culture Conditions

In one embodiment, the disclosure is directed to a cell culturing process comprising: culturing host/inoculum cells, which express a protein of interest, in growth factor/protein/peptide free media; and adding one or more growth factors to the production cell culture.

A growth factor is a naturally occurring substance capable of stimulating cellular growth, and cellular differentiation. Usually it is a protein or a steroid hormone. Growth factors are important for regulating a variety of cellular processes. Growth factors typically act as signaling molecules for cells. They often promote cell differentiation and maturation, which varies between growth factors.

Growth factors include but are not limited to, insulin (GIBCO® rHu AOF Insulin, Biocon), platelet-derived growth factor (PDGF), basic fibroblast growth factor (bFGF), epidermal growth factor (EGF) (LONG®EGF, RepliGen Bioprocessing), insulin-like growth factor (IGF) (LONGOR31GF-1, RepilGen Bioprocessing), transforming growth factor alpha (TGF-α) (LONG®TGF-α, RepliGen Bioprocessing), erythropoietin, steroids, serum, nerve growth factor (NGF), fibroblast growth factor (FGF) and colony-stimulating factor (CSF). The compounds are readily available from the listed sources, or readily obtainable through means known to one of skill in the art.

In one embodiment the growth factors include but are not limited to insulin and/or insulin-like growth factor (IGF).

In one embodiment, growth factor is added at inoculation or may be a component of the basal medium. Inoculation takes place on day 0.

In one embodiment, growth factor is added at a time after inoculation, *i.e.*, it is not present in the basal medium and not present at inoculation. In one specific embodiment, growth factor is added on day 1 of the culture or later.

Growth factor may be added to the cell culture one time, two times, three times, or any number of times during the specified time period. One or more growth factors may be used in conjunction. That is, any given single addition of a growth factor may include the addition of one or more other growth factors. Similarly, if there is more than one addition of a growth factor, different growth factors may be added at the different additions. Additional compounds and substances, including growth factor, may be added to the culture before, with or after the addition of growth factor - either during or not during the specified time period. In a specific embodiment, growth factor is added with the basal medium after inoculation. In another embodiment, growth factor is added with the feed medium. In a specific embodiment, growth factor is added with the basal medium after inoculation and with the feed medium. In another specific embodiment, one growth factor is added.

Growth factor may be added to the cell culture by any means. Means of adding growth factor include, but are not limited to, dissolved in water, dissolved in an acid, dissolved in basal medium, dissolved in feed medium, dissolved in a suitable medium, in the form in which it is obtained or any combination thereof.

In one embodiment, insulin is added as a solution where the insulin is dissolved in 1 M HCL that is then diluted with water for further use (*i.e.,* such as adding insulin to the feed medium).

In one embodiment, growth factor is added to bring the concentration in the culture to an appropriate level. As non-limiting examples, insulin is added to maintain a concentration of $1\mu g/L$ - 10mg/L. In another embodiment, insulin is added to maintain a concentration of $4\mu g/L$ - 10mg/L. In still another embodiment insulin is added to maintain a concentration of $40\mu g/L$ - 10mg/L.

In one embodiment, growth factor is added to the basal medium in an amount of about $1\mu g/L$ to 10 mg/L. In another embodiment, growth factor is added to the basal medium in an amount of about $1\mu g/L$ to 1 mg/L. In a non-limiting example, insulin may be added to the basal medium in an amount of about $2\mu g/L$ to 100 $\mu g/L$, insulin may be added to the basal medium in an amount of about $2\mu g/L$, $10\mu g/L$, 100 $\mu g/L$ or 1mg/mL. Other non-limiting examples include, the addition of IGF, bFGF and/or PDGF may be added to the basal medium in an amount of about $4\mu g/L$.

In one embodiment, growth factor is added to the feed medium in an amount of about $1\mu g/L$ to 10 mg/L. In another embodiment, growth factor is added to the feed medium in an amount that is from 5 fold to 10 fold that of the basal medium. In a non-limiting example, insulin may be added to the feed medium in an amount of about 10 mg/L and/or insulin-like growth factor may be added to the feed medium in an amount of about 40 $\mu g/L$.

The culture may be run for any length of time after addition of growth factor. The culture run time may be determined by one of skill in the art, based on relevant factors such as the quantity and quality of recoverable protein, and the level of contaminating cellular species (e.g., proteins and DNA) in the supernatant resulting from cell lysis, which will complicate recovery of the protein of interest.

In particular embodiments of the cell culturing process and method of increasing cell viability, growth factor is added at a time after inoculation that is before the beginning of the initial death phase. Alternatively, growth factor is added at a time after inoculation that is during the initial growth phase, or growth factor is added during the second half the initial growth phase, or growth factor is added on or about the end of the initial growth phase.

The initial growth phase refers to the growth phase that is observed in the absence of the specified addition of growth factor. The initial death phase refers to the death phase that is observed in the absence of the specified addition of growth factor.

The initial growth phase may end when the initial death phase begins, or there may be a stationary phase of any length between the initial growth phase and the initial death phase.

For example, in a cell culture in which the initial growth phase is from day 0 to day 6 and the initial death phase begins on day 7, in a particular embodiment growth factor is added at a time after inoculation and before day 7. In a specific embodiment, growth factor is added after inoculation and by day 6. In a specific embodiment, growth factor is added between days 1 and 6. In another specific embodiment, growth factor is added with the feed medium on days 3-6. In other specific embodiments, growth factor is added on about day 2, or on day 2.

It has been found (see Figures 9 and 10) that when carrying out the processes disclosed herein the viability of the cell culture is prolonged. A condition, such as addition of growth factor, causes prolonged cell viability if cell viability in the culture is higher for a period of time in the presence of the condition than in the absence of the condition.

Thus, in other embodiments, the disclosure is directed to a cell culturing process for prolonging cell viability in a culture comprising: culturing host cells, which express a protein of interest, in growth factor/protein/peptide free media; and adding growth factor to the cell culture; wherein the end cell viability of the cell culture is prolonged compared to end cell viability of cells grown in media comprising growth factor/protein/peptide.

Run times of cell culture processes, particularly non-continuous processes, are usually limited by the remaining viable cell density, which decreases during the death phase. Longer run times may allow higher product titers to be achieved. Product quality concerns also offer a motivation for reducing death rate as the presence of cell debris and the contents of dead cells in the culture can negatively impact on the ability to isolate and/or purify the protein product at the end of the culturing run.

It has been found (see Figures 9, 10 and 12) that prolonged cell viability achieved by carrying out the processes disclosed herein results in increased protein titer.

Thus, in other embodiments, the disclosure is directed to a cell culturing process for increasing protein production in a culture comprising: culturing host cells, which express a protein of interest, in growth factor/protein/peptide free media; and adding growth factor to the cell culture; wherein the protein titer is increased compared to protein titier of cells grown in media comprising growth factor/protein/peptide.

Techniques and Procedures Relating to Protein Purification and Analysis

In the culturing methods encompassed by the present invention, the protein produced by the cells is typically collected, recovered, isolated, and/or purified, or substantially purified, as desired, at the end of the total cell culture period using isolation and purification methods as known and practiced in the art. Preferably, protein that is secreted from the cultured cells is isolated from the culture medium or supernatant; however, protein can also be recovered from the host cells, *e.g.,* cell lysates, using methods that are known.

Illustratively, for protein recovery, isolation and/or purification, the cell culture medium or cell lysate is centrifuged to remove particulate cells and cell debris. The desired polypeptide product is isolated or purified away from contaminating soluble proteins and polypeptides by suitable purification techniques. The following procedures provide exemplary, yet nonlimiting purification methods for proteins: separation or fractionation on immunoaffinity or ion-exchange columns; ethanol precipitation; reverse phase HPLC; chromatography on a resin, such as silica, or cation exchange resin, e.g., DEAE; chromatofocusing; SDS-PAGE; ammonium sulfate precipitation; gel filtration using, *e.g.,* SEPHADEX® G-75, SEPHAROSE®; protein A SEPHAROSE® chromatography for removal of immunoglobulin contaminants; and the like. Other additives, such as protease inhibitors (*e.g.,* PMSF or proteinase K) can be used to inhibit proteolytic degradation during purification. It will be understood by the skilled practitioner that purification methods for a given polypeptide of interest may require modifications which allow for changes in the polypeptide expressed recombinantly in cell culture.

Cells, Proteins and Cell Cultures

In the cell culture processes or methods of this invention, the cells can be maintained in a variety of cell culture media. *i.e.*, basal culture media, as conventionally known in the art. For example, the methods are applicable for use with large volumes of cells maintained in cell culture medium, which can be supplemented with nutrients and the like. Typically, "chemically defined cell culturing medium" (also called "chemically defined medium") is a term that is understood by the practitioner in the art and is known to refer to a nutrient solution in which cells, preferably animal or mammalian cells, are grown and which generally provides at least one or more components from the following: an energy source (usually in the form of a carbohydrate such as glucose); all essential amino acids, and generally the twenty basic amino acids, plus cysteine; vitamins and/or other organic compounds typically required at low concentrations; lipids or free fatty acids, *e.g.,* linoleic acid; and trace elements, *e.g.,* inorganic compounds or naturally occurring elements that are typically required at very low concentrations, usually in the micromolar range. Cell culture medium can also be supplemented to contain a variety of optional components, such as salts, *e.g.,* calcium, magnesium and phosphate, and buffers, *e.g.,* HEPES; nucleosides and bases, *e.g.,* adenosine, thymidine, hypoxanthine; antibiotics, *e.g.,* gentamycin; and cell protective agents, *e.g.,* a PLURONIC® polyol (PLURONIC® F68).

One embodiment of the invention is a cell culture process utilizing a chemically defined cell culture media that is free of growth factors, proteins and peptides, *e.g.,* insulin, transferrin, epidermal growth factor, serum, hydrolyzed protein, hormones, bFGF, IGF, PDGF and free of products or ingredients of animal origin.

As is appreciated by the practitioner, animal or mammalian cells are cultured in a medium suitable for the particular cells being cultured and which can be determined by the person of skill in the art without undue experimentation. Commercially available media can be utilized and include, for example, Minimal Essential Medium (MEM, Sigma, St. Louis, MO); Ham's F10 Medium (Sigma); Dulbecco's Modified Eagles Medium (DMEM, Sigma); RPMI-1640 Medium (Sigma); HYCLONE® cell culture medium (HyClone, Logan, UT); and chemically-defined (CD) media, which are formulated for particular cell types, *e.g.,* CD-CHO Medium (Invitrogen, Carlsbad, CA). EX-CELL® CD-CHO (SAFC, Saint Louis).

To the foregoing exemplary media can be added the above-described supplementary components or ingredients, including optional components, in appropriate concentrations or amounts, as necessary or desired, and as would be known and practiced by those having in the art using routine skill.

In addition, cell culture conditions suitable for the methods of the present invention are those that are typically employed and known for batch, fed-batch, or continuous culturing of cells, with attention paid to pH, *e.g.,* about 6.5 to about 7.5; dissolved oxygen ($O_2$), *e.g.,* between about 5-90% of air saturation and carbon dioxide($CO_2$), agitation and

humidity, in addition to temperature. As an illustrative, yet nonlimiting, example, a suitable cell culturing medium for the fed-batch processes of the present invention comprises a modified CD-CHO Medium (Invitrogen, Carlsbad, CA). A feeding medium can also be employed, such as modified eRDF medium (Invitrogen, Carlsbad, CA). One embodiment of the disclosure utilizes a feeding medium also containing a growth factor, *e.g.,* insulin.

Animal cells, mammalian cells, cultured cells, animal or mammalian host cells, host cells, recombinant cells, recombinant host cells, and the like, are all terms for the cells that can be cultured according to the processes of this disclosure.. Such cells are typically cell lines obtained or derived from mammals and are able to grow and survive when placed in either monolayer culture or suspension culture in medium containing appropriate nutrients.

Numerous types of cells can be cultured according to the methods of the present disclosure. The cells are typically animal or mammalian cells that can express and secrete, or that can be molecularly engineered to express and secrete, large quantities of a particular protein of interest, into the culture medium. It will be understood that the protein produced by a host cell can be endogenous or homologous to the host cell. Alternatively, the protein is heterologous, *i.e.,* foreign, to the host cell, for example, a human protein produced and secreted by a Chinese hamster ovary (CHO) host cell. In one embodiment, mammalian proteins, *i.e.,* those originally obtained or derived from a mammalian organism, are attained by the methods the present invention and are preferably secreted by the cells into the culture medium.

Proteins of interest may include fusion proteins and polypeptides, chimeric proteins and polypeptides, as well as fragments or portions, or mutants, variants, or analogues of any of the aforementioned proteins and polypeptides are also included among the suitable proteins, polypeptides and peptides that can be produced by the methods of the present invention.

Nonlimiting examples of animal or mammalian host cells suitable for harboring, expressing, and producing proteins for subsequent isolation and/or purification include Chinese hamster ovary cells (CHO), such as CHO-K1 (ATCC CCL-61), DG44 (Chasin et al., Som. Cell Molec. Genet., 12:555-556 (1986); and Kolkekar et al., Biochemistry, 36:10901-10909 (1997)), CHO-K1 Tet-On cell line (Clontech), CHO designated ECACC 85050302 (CAMR, Salisbury, Wiltshire, UK), CHO clone 13 (GEIMG, Genova, IT), CHO clone B (GEIMG, Geneva, IT), CHO-K1/SF designated ECACC 93061607 (CAMR, Salisbury, Wiltshire, UK), RR-CHOK1 designated ECACC 92052129 (CAMR, Salisbury, Wiltshire, UK), dihydrofolate reductase negative CHO cells (CHO/-DHFR, Urlaub et al., Proc. Natl. Acad. Sci. USA, 77:4216 (1980)), dp12.CHO cells (U.S. Patent No. 5,721,121) and FUT8 (alpha-1,6-fucosyltransferase) knockout CHO cell line, Ms704 (Naoko Yamane-Ohnuki et al., published online August 6, 2004 in Wiley InterScience (www.interscience.wiley.com). DOI: 10.1002/bit.20151); monkey kidney CV1 cells transformed by SV40 (COS cells, COS-7, ATCC® CRL-1651); human embryonic kidney cells (*e.g.,* 293 cells, or 293 cells subcloned for growth in suspension culture, Graham et al., J. Gen. Virol., 36:59 (1977)); baby hamster kidney cells (BHK, ATCC® CCL-10); monkey kidney cells (CV1, ATCC® CCL-70); African green monkey kidney cells (VERO-76, ATCC® CRL-1587; VERO, ATCC® CCL-81); mouse sertoli cells (TM4, Mather, Biol. Reprod., 23:243-251 (1980)); human cervical carcinoma cells (HELA, ATCC® CCL-2); canine kidney cells (MDCK, ATCC® CCL-34); human lung cells (W138, ATCC® CCL-75); human hepatoma cells (HEP-G2, HB 8065); mouse mammary tumor cells (MMT 060562, ATCC® CCL-51); buffalo rat liver cells (BRL 3A, ATCC® CRL-1442); TRI cells (Mather, Ann. NY Acad. Sci., 383:44-68 (1982)); MCR 5 cells; FS4 cells. According to the invention the cells are CHO cells, such as CHO/-DHFR cells and CHO/-GS cells.

The cells suitable for culturing in the methods and processes of the present invention can contain introduced, *e.g.,* via transformation, transfection, infection, or injection, expression vectors (constructs), such as plasmids and the like, that harbor coding sequences, or portions thereof, encoding the proteins for expression and production in the culturing process. Such expression vectors contain the necessary elements for the transcription and translation of the inserted coding sequence. Methods which are well known to and practiced by those skilled in the art can be used to construct expression vectors containing sequences encoding the produced proteins and polypeptides, as well as the appropriate transcriptional and translational control elements. These methods include *in vitro* recombinant DNA techniques, synthetic techniques, and *in vivo* genetic recombination. Such techniques are described in Sambrook, J. et al., Molecular Cloning, A Laboratory Manual, Cold Spring Harbor Press, Plainview, NY (1989) and in Ausubel, F.M. et al., Current Protocols in Molecular Biology, John Wiley & Sons, New York, NY (1989).

Control elements, or regulatory sequences, are those non-translated regions of the vector, e.g., enhancers, promoters, 5' and 3' untranslated regions, that interact with host cellular proteins to carry out transcription and translation. Such elements can vary in their strength and specificity. Depending on the vector system and host cell utilized, any number of suitable transcription and translation elements, including constitutive and inducible promoters, can be used. In mammalian cell systems, promoters from mammalian genes or from mammalian viruses are preferred. The constructs for use in protein expression systems are designed to contain at least one promoter, an enhancer sequence (optional, for mammalian expression systems), and other sequences as necessary or required for proper transcription and regulation of gene expression (*e.g.,* transcriptional initiation and termination sequences, origin of replication sites, polyadenylation sequences).

As will be appreciated by those skilled in the art, the selection of the appropriate vector, *e.g.,* plasmid, components for proper transcription, expression, and isolation of proteins produced in eukaryotic (*e.g.,* mammalian) expression systems is known and routinely determined and practiced by those having skill in the art. The expression of proteins by the cells cultured in accordance with the methods of this invention can placed under the control of promoters such as viral promoters, *e.g.,* cytomegalovirus (CMV), Rous sarcoma virus (RSV), phosphoglycerol kinase (PGK), thymidine kinase (TK), or the α-actin promoter. Further, regulated promoters confer inducibility by particular compounds or molecules. Also, tissue-specific promoters or regulatory elements can be used (Swift, G. et al., Cell, 38:639-646 (1984)), if necessary or desired.

Expression constructs can be introduced into cells by a variety of gene transfer methods known to those skilled in the art, for example, conventional gene transfection methods, such as calcium phosphate co-precipitation, liposomal transfection, microinjection, electroporation, and infection or viral transduction. The choice of the method is within the competence of the skilled practitioner in the art. It will be apparent to those skilled in the art that one or more constructs carrying DNA sequences for expression in cells can be transfected into the cells such that expression products are subsequently produced in and/or obtained from the cells.

In a particular aspect, mammalian expression systems containing appropriate control and regulatory sequences are preferred for use in protein expressing mammalian cells of the present invention. Commonly used eukaryotic control sequences for use in mammalian expression vectors include promoters and control sequences compatible with mammalian cells such as, for example, the cytomegalovirus (CMV) promoter (CDM8 vector) and avian sarcoma virus (ASV), (πLN). Other commonly used promoters include the early and late promoters from Simian Virus 40 (SV40) (Fiers et al., Nature, 273:113 (1973)), or other viral promoters such as those derived from polyoma, Adenovirus 2, and bovine papilloma virus. An inducible promoter, such as hMTII (Karin et al., Nature, 299:797-802 (1982)) can also be used.

Examples of expression vectors suitable for eukaryotic host cells include, but are not limited to, vectors for mammalian host cells (*e.g.,* BPV-1, pHyg, pRSV, pSV2, pTK2 (Maniatis); pIRES (Clontech); pRc/CMV2, pRc/RSV, pSFV1 (Life Technologies); pVPakc Vectors, pCMV vectors, pSG5 vectors (Stratagene), retroviral vectors (*e.g.,* pFB vectors (Stratagene)), pcDNA-3 (Invitrogen), adenoviral vectors; Adeno-associated virus vectors, baculovirus vectors, yeast vectors (*e.g.,* pESC vectors (Stratagene)), or modified forms of any of the foregoing. Vectors can also contain enhancer sequences upstream or downstream of promoter region sequences for optimizing gene expression.

A selectable marker can also be used in a recombinant vector (*e.g.,* a plasmid) to confer resistance to the cells harboring (preferably, having stably integrated) the vector to allow their selection in appropriate selection medium. A number of selection systems can be used, including but not limited to, the Herpes Simplex Virus thymidine kinase (HSV TK), (Wigler et al., Cell, 11:223 (1977)), hypoxanthine-guanine phosphoribosyltransferase (HGPRT), (Szybalska et al., Proc. Natl. Acad. Sci. USA, 48:202 (1992)), and adenine phosphoribosyltransferase (Lowy et al., Cell, 22:817 (1980)) genes, which can be employed in tk-, hgprt-, or aprt- cells (APRT), respectively.

Anti-metabolite resistance can also be used as the basis of selection for the following nonlimiting examples of marker genes: dhfr, which confers resistance to methotrexate (Wigler et al., Proc. Natl. Acad. Sci. USA, 77:357 (1980); and O'Hare et al., Proc. Natl. Acad. Sci. USA, 78:1527 (1981)); glutamine synthase (GS), which confers resistance to methionine sulphoximine; gpt, which confers resistance to mycophenolic acid (Mulligan et al., Proc. Natl. Acad. Sci. USA, 78:2072 (1981)); neo, which confers resistance to the aminoglycoside G418 (Clinical Pharmacy, 12:488-505; Wu et al., Biotherapy, 3:87-95 (1991); Tolstoshev, Ann. Rev. Pharmacol. Toxicol., 32:573-596 (1993); Mulligan, Science, 260:926-932 (1993); Anderson, Ann. Rev. Biochem., 62:191-121 (1993); TIB TECH, 11(5): 155-215 (May 1993); and hygro, which confers resistance to hygromycin (Santerre et al., Gene, 30:147 (1984)). Methods commonly known in the art of recombinant DNA technology can be routinely applied to select the desired recombinant cell clones, and such methods are described, for example, in Ausubel et al., eds., Current Protocols in Molecular Biology, John Wiley & Sons, NY (1993); Kriegler, Gene Transfer and Expression, A Laboratory Manual, Stockton Press, NY (1990); in Chapters 12 and 13, Dracopoli et al., eds, Current Protocols in Human Genetics, John Wiley & Sons, NY (1994); Colberre-Garapin et al., J. Mol. Biol., 150:1 (1981).

In addition, the expression levels of an expressed protein molecule can be increased by vector amplification (for a review, see Bebbington, C.R. et al., Chapter 8: "The use of vectors based on gene amplification for the expression of cloned genes in mammalian cells", Glover, D.M., ed., DNA Cloning, Vol. 3: A Practical Approach, pp. 163-188, IRL Press Limited, publ. (1987)). When a marker in the vector system expressing a protein is amplifiable, an increase in the level of inhibitor present in the host cell culture will increase the number of copies of the marker gene. Since the amplified region is associated with the protein-encoding gene, production of the protein will concomitantly increase (Crouse et al., Mol. Cell. Biol., 3:257 (1983)).

Vectors which harbor glutamine synthase (GS) or dihydrofolate reductase (DHFR) encoding nucleic acid as the selectable markers can be amplified in the presence of the drugs methionine sulphoximine or methotrexate, respectively. An advantage of glutamine synthase based vectors is the availability of cell lines (*e.g.,* the murine myeloma cell line, NSO) which are glutamine synthase negative. Glutamine synthase expression systems can also function in glutamine synthase expressing cells (*e.g.,* CHO cells) by providing additional inhibitor to prevent the functioning of the endogenous gene.

Vectors that express DHFR as the selectable marker include, but are not limited to, the pSV2-dhfr plasmid (Subramani et al., Mol. Cell. Biol. 1:854 (1981)). Vectors that express glutamine synthase as the selectable marker include, but are not limited to, the pEE6 expression vector described in Stephens et al., Nucl. Acids. Res., 17:7110 (1989). A glutamine synthase expression system and components thereof are detailed in PCT publications: WO 87/04462; WO 86/05807; WO 89/01036; WO 89/10404; and WO 91/06657. In addition, glutamine synthase expression vectors that can be used in accordance with the present invention are commercially available from suppliers, including, for example, Lonza Biologics, Inc. (Portsmouth, NH).

Types of Cell Cultures

For the purposes of understanding, yet without limitation, it will be appreciated by the skilled practitioner that cell cultures and culturing runs for protein production can include three general types; namely, continuous culture, batch culture and fed-batch culture. In a continuous culture, for example, fresh culture medium supplement (*i.e.,* feeding medium) is provided to the cells during the culturing period, while old culture medium is removed daily and the product is harvested, for example, daily or continuously. In continuous culture, feeding medium can be added daily and can be added continuously, *i.e.,* as a drip or infusion. For continuous culturing, the cells can remain in culture as long as is desired, so long as the cells remain alive and the environmental and culturing conditions are maintained.

In batch culture, cells are initially cultured in medium and this medium is neither removed, replaced, nor supplemented, *i.e.,* the cells are not "fed" with new medium, during or before the end of the culturing run. The desired product is harvested at the end of the culturing run.

For fed-batch cultures, the culturing run time is increased by supplementing the culture medium with fresh medium during the run, *i.e.,* the cells are "fed" with new medium ("feeding medium") during the culturing period. Fed-batch cultures can include various feeding regimens and times, for example, daily, every other day, every two days, etc., more than once per day, or less than once per day, and so on. Further, fed-batch cultures can be fed continuously with feeding medium.

The desired product is then harvested at the end of the culturing/production run.

One embodiment of the present disclosure embraces fed-batch cell cultures in which growth factors such as insulin and/or IGF is added at a time after inoculation.

According to the present invention, cell culture can be carried out, and proteins can be produced by cells, under conditions for the large or small scale production of proteins, using culture vessels and/or culture apparatuses that are conventionally employed for animal or mammalian cell culture. As is appreciated by those having skill in the art, tissue culture dishes, T-flasks and spinner flasks are typically used on a laboratory scale. For culturing on a larger scale, (*e.g.,* 500 L, 5000 L, and the like, for example, as described in commonly-assigned U.S. Patent Nos. 7,541,164 and 7,332,303, and U.S. Patent Application 2009 0252749 A1 (Serial No. 12/086,786), filed December 19,2006) procedures including, but not limited to, a fluidized bed bioreactor, a hollow fiber bioreactor, roller bottle culture, or stirred tank bioreactor systems can be used. Microcarriers may or may not be used with the roller bottle or stirred tank bioreactor systems. The systems can be operated in a batch, continuous, or fed-batch mode. In addition, the culture apparatus or system may or may not be equipped with a cell separator using filters, gravity, centrifugal force, and the like.

Phases of Cell Culture and Associated Parameters

The term "inoculation" refers to the addition of cells to starting medium to begin the culture.

The "growth phase" of a culture is the phase during which the viable cell density at any time point is higher than at any previous time point.

The "stationary phase" of a culture is the phase during which the viable cell density is approximately constant (*i.e.,* within measuring error) over a time period of any length.

The "death phase" of a culture is the phase that comes after the growth phase or after the growth phase and the stationary phase, and during which the viable cell density at any time point is lower than at any previous time point during that phase.

In one embodiment of the invention, the culture medium is supplemented ("fed") during the production phase to support continued protein production, particularly in an extended production phase, and to attain ample quantities of high quality protein product. Feeding can occur on a daily basis, or according to other schedules to support cell viability and protein production.

The culturing process according to the present invention may result in more viable cell survival until the end of the culturing period. Accordingly, in some embodiments, the more cells that survive, the more cells that are producing the desired product. This, in turn, results in a greater accumulated amount of the product at the end of the culturing process, with the rate of protein production by individual cells, *i.e.,* cell specific productivity, remaining the same. Cell specific productivity or cell specific rate, as known in the art, typically refers to the specific expression rate of product produced per cell, or per measure of cell mass or volume. Cell specific productivity is measured in grams of protein produced per cell per day, for example, and can be measured according to an integral method involving the following formulae:

$$dP/dt = q_p X, \text{ or}$$

$$P = q_p \int_0^t Xdt$$

where $q_p$ is the cell specific productivity constant; X is the number of cells or cell volume, or cell mass equivalents; and dP/dt is the rate of protein production. Thus, qp can be obtained from a plot of product concentration versus time integral of viable cells ($\int_0^t$ Xdt "viable cell days"). According to this formula, when the amount of protein product produced is plotted against the viable cell days, the slope is equivalent to the cell specific rate. Viable cells can be determined by several measures, for example, biomass, $O_2$ uptake rate, lactase dehydrogenase (LDH), packed cell volume or turbidity. (*e.g.,* U.S. Patent No. 5,705,364 to Etcheverry, T. et al.)

Production of aCD40L Fusion Protein by the Culturing Methods of the Present Invention

In other embodiments encompassed by the present invention, the cell culture methods of the invention are utilized to produce a soluble aCD40L fusion protein, as exemplified by **Clone A** and **Clone B** described herein.

Soluble aCD40L is an antibody polypeptide that specifically binds human CD40L. The antibody polypeptide is a domain antibody (dAbs) comprising a variable domain. Soluble aCD40L is useful in the treatment of diseases involving CD40L activation, such as autoimmune diseases. U.S. Patent Application Serial No. 61/655,110, filed June 4, 2012 describes the aCD40L dAb.

In a another embodiment, soluble aCD40L is produced by recombinantly engineered host cells. According to the invention, the soluble aCD40L protein is recombinantly produced by CHO cells transfected with a vector containing the DNA sequence encoding soluble aCD40L. The soluble aCD40L fusion protein is produced in high quantity and when cultured in accordance with the processes of this invention. The invention affords the production of high levels of recoverable protein product, e.g., soluble aCD40L protein product as shown in Example 3, 4 and 5; Figures 4, 6 and 9.

Production of a Myostatin Fusion Protein by the Culturing Methods of the Present Invention

In other embodiments encompassed by the present invention, the cell culture methods of the invention are utilized to produce a soluble myostatin fusion protein, as exemplified by **Clone C** described herein.

In another embodiment, myostatin fusion protein is produced by recombinantly engineered host cells. According to the invention, the myostatin fusion protein can be recombinantly

produced by CHO cells transfected with a vector containing the DNA sequence encoding the myostatin fusion protein. The myostatin fusion protein is produced in high quantity and when cultured in accordance with the processes of this invention. The invention affords the production of high levels of recoverable myostatin fusion protein product as shown in Example 3 and 4; Figures 7 and 10.

Production of CTLA4Ig Fusion Protein by the Culturing Methods of the Present Invention

In other embodiments encompassed by the present invention, the cell culture methods of the invention are utilized to produce a soluble CTLA4Ig fusion protein, as exemplified by **Clone D** described herein. CTLA4Ig is a soluble fusion protein that consists of the extracellular domain of human cytotoxic T-lymphocyte-associated antigen 4 (CTLA-4) linked to the modified Fc (hinge, CH2, and CH3 domains) portion of human immunoglobulin G1(IgG1). CTLA4 is indicated for reducing signs and symptoms, inducing major clinical response, inhibiting the progression of structural damage, and improving physical function in adult patients with moderately to severely active rheumatoid arthritis. The CTLA4Ig fusion protein can be recombinantly produced by CHO cells transfected with a vector containing the DNA sequence encoding CTLA4Ig as described in U.S. Patent No. 5,844,095 to Linsley, P.S. et al.

In another embodiment, soluble CTLA4Ig is produced by recombinantly engineered host cells. According to the invention, the soluble CTLA4Ig protein can be recombinantly produced by CHO cells transfected with a vector containing the DNA sequence encoding soluble CTLA4Ig. The soluble CTLA4Ig fusion protein is produced in high quantity and when cultured in accordance with the processes of this invention. The invention affords the production of high levels of recoverable soluble CTLA4Ig protein product as shown in Example 3 and 4; Figures 8 and 12.

EXAMPLES

The following Examples set forth specific aspects of the invention to illustrate the invention and provide a description of the present methods for those of skill in the art. The Examples should not be construed as limiting the invention, as the Examples merely provide specific methodology and exemplification that are useful in the understanding and practice of the invention and its various aspects.

Examples 1-5 as set forth below describe experiments relating to cell culture processes involving the culturing process of the invention with and without growth factors.

Example 1

CHO Cells were Able to Grow Under GF-Free Condition

1. Thaw a new vial of cells in early passage and culture in platform medium (with 1 or 10 mg/L insulin) for 2 passages.

2. At passage 3, transfer the cells to basal media without insulin, and keep splitting every 3 to 4 days at a density of $0.6 \times 10^6$ cells/ml.

3. In the first few passages under insulin free conditions, cell growth may significantly slow down and viability may drop (~90%). Meanwhile, ammonium production may be increased due to insufficient glucose uptake and oxidation of amino acids as an energy source. As a result, pH in flasks may increase. $CO_2$ level may need to be adjusted/increased accordingly to control pH in a range of 7.0-7.3. This is very important to maintain the cells in a healthy state.

4. In general, spent medium carried into a new passage should be less than 30%. In case cell growth is too slow to satisfy the criterion, cells could be centrifuged down and transferred at a desired number to a new flask which contains 30% spent medium and 70% fresh medium.

5. When cells are fully adapted, cell growth will recover and viability will come back to over 95%. It may take up to 7~9 passages to adapt the cells to insulin free condition depending on the specific clone.

6. Cell culture parameters: shaking speed: 150 rpm; shake flask: 250 ml or 500 ml baffled shake flasks (Coming Inc.) with 100 ml or 200 ml working volume; temperature: 37 °C; $CO_2$: 6% in general, may be adjusted to control pH at 7.0~7.3 (up to 8% $CO_2$).

Figure 1 and Figure 2 show the adaption of Clone A and Clone B, respectively, to growth in medium without growth factors. Similar results were seen for other Clones C and D.

Example 2

Removal of Insulin did not Drastically Impact the mTOR Pathway

The purpose of this study was to use antibody array to compare the phosphorylation/expression level of proteins involved in mTOR in cell grown with and without growth factor, insulin, thereby demonstrating that from a molecular and cell biology perspective that cells are able to grow under GF-free condition.

Antibody Array Sample Preparation

Clone A cultured under insulin-free or insulin containing basal medium. $5 \times 10^6$ viable cells were sampled for each condition on day 3. Cells were centrifuged down at 500g for 5 min and at 4 °C. Cells were washed with 10 ml ice cold 1xPBS, and centrifuged down at 500g for 5 min and at 4 °C. Cells were always kept on ice or 4 °C during sample processing. After dumping the PBS, cells were frozen at -70 °C immediately and stored at -70 °C till antibody array analysis

Antibody Array Protocol

Protein Extraction

Wash the cells with ice cold 1X PBS. Add Lysis Beads and Extraction Buffer to the sample. Mix rigorously by vortexing for 30 seconds. Incubate the mixture on ice for 10 minutes. Repeat vortexing for 30 seconds at 10-minute intervals for 60 minutes. Incubate the mixture on ice between vortexing. Centrifuge the mixture at 10,000 x g for 20 minutes at 4 °C. Transfer the supernatant to a clean tube. Use spin columns to change the buffer in the supernatant to Labeling Buffer. Measure protein concentration. Note: phosphatase inhibitors are included in Extraction Buffer and Labeling Buffer.

Protein Labeling

Add 100 μL of DMF to 1 mg of Biotin Reagent to give a final concentration of 10 μg/μL. Add Labeling Buffer to the protein sample to bring the volume to 75 μL. Add 3 μL of the Biotin/DMF to the protein sample with Labeling Buffer. Mix and incubate at room temperature for two hours with mixing. Add 35 μL of Stop Reagent. Incubate for 30 minutes at room temperature with mixing.

Coupling

Blocking: Submerge Antibody Microarray in Blocking Buffer. Shake for 40 minutes at room temperature. Rinse the slide with MILLI-Q® grade water.

Incubate the slide in Coupling Chamber with 85μg of labeled protein sample in 6 mL Coupling Solution on an orbital shaker for 2 hours at room temperature. Remove the slide from the coupling chamber. Wash the slide three times with fresh Wash Buffer. Rinse extensively with DI water.

Detection

Add 30 μl of Cy3-Streptavidin (1 mg/ml) to the 60-ml bottle containing Detection Buffer. Submerge the slide in 30 ml of Cy3-Streptavidin solution. Incubate on an orbital shaker for 45 minutes at room temperature in the dark. Wash the slide three times with fresh Wash Buffer. Rinse extensively with DI water. Dry the slide with compressed nitrogen. Scan on Axon GenePix Array Scanner.

Assay Data

For each spot on the array, median spot intensity is extracted from array image. Using the median intensity, determine the average signal of the replicate spots for each antibody. The data is labeled as *Average Signal of Replicate Spots on the Array*. The *CV of the Replicates on the Array* is the coefficient of variation of the replicate spots for each antibody. For normalization, within each array slide the mean value of the Average Signal of all antibodies in the array is determined. This value is presented as *Mean Signal.*

$$\text{Normalized data} = \text{Average Signal of Replicate Spots} / \text{Mean Signal}$$

Using the normalized data, determine the fold change between control and treated samples.

$$\textit{Signal Fold change} = \text{Treated/Control}$$

Example 3

GFs/Proteins/Peptides Free Media Improve Process Performance

Cells: Clone A, B, C and D.

Cell culture parameters in shake flasks:

Shaking speed: 150 rpm; shake flask: 250 ml or 500 ml baffled shake flasks (Corning Inc.) with 100 ml or 200 ml working volume; temperature: 37 °C; $CO_2$: 6% in general.

Cell culture parameters in reactors:

Agitation 120 rpm; 5L reactor with 1.3L initial culture volume; temperature: 37 °C; dissolved oxygen (DO) controlled at 50%; pH at 7.0 to 7.3.

Seeding density: 0.3 or $0.6 \times 10^6$ cells/ml.

Basal: 171 medium for Clone A, B and C with or without insulin; 127G medium for Clone D with or without insulin.

Feed: 154A-1 or 154A-1 derived medium (M154A1B for Clone A and B) with or without insulin.

Feeding strategy: feeding begins on day 3, feed 3.64% initial culture volume till day 14.

Sampling: at designated time points, samples were taken to measure cell density and viability with CEDEX®, titer with HPLC, and high molecular species with size exclusion chromatography (SE).

Example 4

GF Add-back to GF-Free Condition Boosted Cell Growth and Protein Production Cells: Clone B producing aCD40L fusion protein.

Cell culture parameters:

Agitation: 120 rpm; 5L reactor with 1.3L initial culture volume; temperature: 37 °C; dissolved oxygen (DO) controlled at 50%.

Basal: medium with or without insulin.

Feed: with or without insulin and LONG®R3.

INS-Free: no insulin in basal, no insulin and no LONG®R3 in feed.

INS: 1 mg/L insulin in basal, 10 mg/L insulin in feed.

INS Add-back: 1 mg/L insulin and 10 mg/L insulin were added back to basal and feed, respectively. Inoculum was insulin-free cells.

Feeding strategy: feeding begins on day 3, feed 3.64% initial culture volume till day 14.

Sampling: at designated time points, samples were taken to measure cell density and viability with CEDEX®, titer with HPLC, and high molecular species with size exclusion chromatography (SE).

Example 5

GFs/Proteins/Peptides Free Media Improve Process Performance

Cells: Clone 63C2 producing aCD40L fusion protein.

Cell culture parameters:

Agitation: 120 rpm; 5L reactor with 1.3L initial culture volume; temperature: 37 °C; dissolved oxygen (DO) controlled at 50%.

Basal: with or without insulin.

Feed: with or without insulin and LONG®R3.

INS-Free + LR3: 4$\mu$g/L LR3 and 40$\mu$g/L LR3 were added to basal feed medium, respectively. Inoculum was GF-free.

INS + LR3: 4$\mu$g/L LR3 and 40$\mu$g/L LR3 were added to basal with 1mg/L and feed medium with 10mg/L insulin, respectively. Inoculum was in basal with 1 mg/L insulin.

Feeding strategy: feeding begins on day 3, feed 3.64% initial culture volume till day 14.

Sampling: at designated time points, samples were taken to measure cell density and viability with CEDEX®, titer with HPLC, and high molecular species with size exclusion chromatography (SE).

**Claims**

1. A method of decreasing aggregation of a protein of interest during cell culture production phase in CHO cells, comprising:

   a) adapting CHO cells which express the protein of interest to growth factor, protein and peptide free media; and
   b) culturing the adapted CHO cells in growth factor, protein and peptide free media.

2. The method of claim 1, wherein the protein of interest is a soluble aCD40L protein.

3. The method of claim 1, wherein the protein of interest is a soluble myostatin fusion protein.

4. The method of claim 1, wherein the protein of interest is a soluble CTLA4Ig fusion protein.

**Patentansprüche**

1. Verfahren zum Vermindern der Aggregation eines Proteins von Interesse während der Produktionsphase einer Zellkultur in CHO-Zellen, umfassend:

   a) Anpassen von CHO-Zellen, welche das Protein von Interesse exprimieren, an Wachstumsfaktor-, Protein- und Peptid-freie Medien; und
   b) Kultivieren der angepassten CHO-Zellen in Wachstumsfaktor-, Protein- und Peptid-freien Medien.

2. Verfahren nach Anspruch 1, wobei das Protein von Interesse ein lösliches aCD40L-Protein ist.

3. Verfahren nach Anspruch 1, wobei das Protein von Interesse ein lösliches Myostatin-Fusionsprotein ist.

4. Verfahren nach Anspruch 1, wobei das Protein von Interesse ein lösliches CTLA4Ig-Fusionsprotein ist.


**Revendications**

1. Procédé de réduction de l'agrégation d'une protéine d'intérêt pendant la phase de production de culture cellulaire dans des cellules CHO, comprenant:

   a) l'adaptation des cellules CHO qui expriment la protéine d'intérêt à un milieu exempt de facteur de croissance, de protéine et de peptide; et
   b) la mise en culture des cellules CHO adaptées dans un milieu exempt de facteur de croissance, de protéine et de peptide.

2. Procédé selon la revendication 1, dans lequel la protéine d'intérêt est une protéine aCD40L soluble.

3. Procédé selon la revendication 1, dans lequel la protéine d'intérêt est une protéine de fusion myostatine soluble.

4. Procédé selon la revendication 1, dans lequel la protéine d'intérêt est une protéine de fusion CTLA4Ig soluble.

FIG. 1

FIG. 2

FIG. 3

FIG. 4

FIG. 5

EP 2 906 683 B1

FIG. 6

FIG. 7

FIG. 8

EP 2 906 683 B1

FIG. 9

EP 2 906 683 B1

FIG. 10

A

B

C

EP 2 906 683 B1

FIG. 11

EP 2 906 683 B1

FIG. 12

EP 2 906 683 B1

FIG. 13

FIG. 14

EP 2 906 683 B1

FIG. 15

FIG. 16

EP 2 906 683 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5705364 A
- US 5721121 A, Etcheverry, T.
- US 5976833 A, Furukawa, K.
- US 5851800 A, Adamson, L.
- WO 9961650 A
- WO 0065070 A
- WO 0036092 A
- US 4357422 A, Girard
- WO 8704462 A
- WO 8605807 A
- WO 8901036 A
- WO 8910404 A
- WO 9106657 A
- US 7541164 B
- US 7332303 B
- US 20090252749 A1
- US 12086786 B
- US 61655110 A
- US 5844095 A, Linsley, P.S.

### Non-patent literature cited in the description

- **ROESSLER et al.** *Enzyme and Microbial Technology,* 1996, vol. 18, 423-427
- **ZHANGI et al.** *Biotechnol. Prog.,* 2000, vol. 16, 319-325
- **CHIRINO et al.** *Nat. Biotechnol.,* 2004, vol. 22, 1383-1391
- **CROMWELL et al.** *AAPSJ.,* 2006, vol. 8, E572-E579
- **CROMWELL et al.** *AAPS J.,* 2006, vol. 8, E572-E579
- **THOMAS et al.** *Exp. Gerontol.,* 2001, vol. 36, 1519-1526
- **CUOZZO et al.** *Nat. Cell Biol.,* 1999, vol. 1, 130-135
- **CHAKRAVARTHI et al.** *J. Biol. Chem.,* 2004, vol. 279, 39872-39879
- **GOMEZ et al.** *Biotechnol. Bioeng.,* 2012, vol. 109, 125-136
- **CHASIN et al.** *Som. Cell Molec. Genet.,* 1986, vol. 12, 555-556
- **KOLKEKAR et al.** *Biochemistry,* 1997, vol. 36, 10901-10909
- **URLAUB et al.** *Proc. Natl. Acad. Sci. USA,* 1980, vol. 77, 4216
- **GRAHAM et al.** *J. Gen. Virol.,* 1977, vol. 36, 59
- **MATHER.** *Biol. Reprod.,* 1980, vol. 23, 243-251
- **MATHER.** *Ann. NY Acad. Sci.,* 1982, vol. 383, 44-68
- **SAMBROOK, J. et al.** Molecular Cloning, A Laboratory Manual. Cold Spring Harbor Press, 1989
- **AUSUBEL, F.M. et al.** Current Protocols in Molecular Biology. John Wiley & Sons, 1989
- **SWIFT, G. et al.** *Cell,* 1984, vol. 38, 639-646
- **FIERS et al.** *Nature,* 1973, vol. 273, 113
- **KARIN et al.** *Nature,* 1982, vol. 299, 797-802
- **WIGLER et al.** *Cell,* 1977, vol. 11, 223
- **SZYBALSKA et al.** *Proc. Natl. Acad. Sci. USA,* 1992, vol. 48, 202
- **LOWY et al.** *Cell,* 1980, vol. 22, 817
- **WIGLER et al.** *Proc. Natl. Acad. Sci. USA,* 1980, vol. 77, 357
- **O'HARE et al.** *Proc. Natl. Acad. Sci. USA,* 1981, vol. 78, 1527
- **MULLIGAN et al.** *Proc. Natl. Acad. Sci. USA,* 1981, vol. 78, 2072
- *Clinical Pharmacy,* vol. 12, 488-505
- **WU et al.** *Biotherapy,* 1991, vol. 3, 87-95
- **TOLSTOSHEV.** *Ann. Rev. Pharmacol. Toxicol.,* 1993, vol. 32, 573-596
- **MULLIGAN.** *Science,* 1993, vol. 260, 926-932
- **ANDERSON.** *Ann. Rev. Biochem.,* 1993, vol. 62, 191-121
- *TIB TECH,* May 1993, vol. 11 (5), 155-215
- **SANTERRE et al.** *Gene,* 1984, vol. 30, 147
- Current Protocols in Molecular Biology. John Wiley & Sons, 1993
- **KRIEGLER.** Gene Transfer and Expression, A Laboratory Manual. Stockton Press, 1990
- Current Protocols in Human Genetics. John Wiley & Sons, 1994
- **COLBERRE-GARAPIN et al.** *J. Mol. Biol.,* 1981, vol. 150 (1
- A Practical Approach. **BEBBINGTON, C.R. et al.** DNA Cloning. IRL Press Limited, 1987, vol. 3, 163-188
- **CROUSE et al.** *Mol. Cell. Biol.,* 1983, vol. 3, 257
- **SUBRAMANI et al.** *Mol. Cell. Biol.,* 1981, vol. 1, 854

- **STEPHENS et al.** *Nucl. Acids. Res.,* 1989, vol. 17, 7110